# EUROPEAN PATENT APPLICATION

(11) **EP 4 800 698 A1**
(43) Date of publication of application: **02.09.2026**
(21) Application number: 25160482.3
(22) Date of filing: 27.02.2025
(51) Int. Cl.: G16H 20/40, G16H 40/63, A61N 5/00

(54) **USING COMPUTATIONAL METHODS TO EXTRACT ACTIONABLE INSIGHTS FROM CLINICAL RADIOTHERAPY TREATMENT DATABASES**

(71) Applicant: Siemens Healthineers International AG, 6312 Steinhausen (CH)
(72) Inventor: CZEIZLER, Elena, 00390 Helsinki (FI); LAAKSONEN, Hannu, 02130 Espoo (FI); HAKALA, Mikko, 05200 Rajamäki (FI); VERBAKEL, Willem, 1065HR Amsterdam (NL)
(74) Representative: HKW Intellectual Property PartG mbB

(57) **Abstract**

The present invention relates to a computer-implemented method, a computer-implemented apparatus and a computer program product for analyzing a plurality of radiotherapy treatment plans comprising defining a search criterion for identifying the plurality of radiotherapy treatment plans in a data storage, defining a clinical metric to be analyzed based on the identified plurality of radiotherapy treatment plans, requesting, from the data storage, the plurality of radiotherapy treatment plans according to the defined search criterion, retrieving, from the data storage, information associated with the plurality of radiotherapy treatment plans according to the request, analyzing the received information according to the defined clinical metric and outputting a result of the analysis of the information.

## Description

The present invention relates to a computer-implemented method, a computer-implemented apparatus and a computer program product to extract actionable insights from clinical radiotherapy treatment databases.

The increasing number of cancer diagnoses every year increased the demand for modern treatment facilities that are capable of providing an increasing number of patients access to adequate treatment schemes.

A commonly used treatment method for cancer therapy is radiation therapy, e.g., applied by a radiotherapy apparatus, wherein tumor cells are at least locally irradiated by a suitable radiation beam such as, e.g., X-rays, gamma rays, protons, heavy ions, etc. A critical aspect of radiotherapy is the radiation dose required to effectively harm malign tumor cells while ensuring that the radiation dose deployed in healthy tissue is minimized (e.g., to avoid the risk of causing carcinogenic cell defects as a result of the radiation process) while keeping the overall duration (e.g., the total time required to deliver a pre-defined radiation dose to a malign tissue of a patient) of the radiation process as low as possible.

A radiotherapy apparatus may comprise a radiation particle source (e.g., for electrons, protons, heavy ions, X-rays or gamma rays, etc.), an acceleration stage and/or may comprise a gantry for directing the accelerated radiation particles onto a patient.

An actual radiotherapy is often carried out based on a preceding radiotherapy treatment planning during which a dose to be delivered to a patient is determined for a plurality of settings of a radiotherapy apparatus (e.g., various directions under which a radiation beam may impinge onto a patient, an energy of the radiation beam and/or radiation doses which need to be achieved for certain locations of the body of the patient which should be irradiated).

These radiotherapy treatment plans may be stored in a (centralized) database. Further patient data (e.g., demographic information, medical history, outcomes reported during and/or after an applied radiotherapy) may equally be acquired and oftentimes stored to a different database. This data is frequently acquired and stored but seldomly used for further data analysis and/or information retrieval.

This is oftentimes due to a lack of respective data analysis tools which would allow a fast and easy data/information retrieval and due to a lack of knowledge on integrating those tools in an already existing clinical workflow.

This aspect may be seen disadvantageous as, e.g., over the course of time, many parameters associated with a radiotherapy treatment planning may change. For instance, (human) planners may change, a training of the (human) planners may change, a version of the software used for radiotherapy treatment planning may change, a radiation beam setup may change and/or optimization objectives may change over time and/or clinical protocols (such as clinical goals and consensus tradeoffs) may change. Even though the general aim of these changes may be seen in improving the radiotherapy treatment planning and the actual radiotherapy treatment as such, these changes cannot be monitored on individual patient cases. Therefore, it may be difficult to know for a medical staff, a clinic and/or other stakeholders if over time the quality of the radiotherapy treatment plans have improved and have not worsened for certain treatment sites which could be in terms of organs at risk (OAR) protection or in terms of planned target volume (PTV) coverage.

What is more, the persistent trend to implement artificial intelligence models in medical diagnostics and radiotherapy treatment plannings requires large amounts of training data to facilitate a training of the artificial intelligence such that the trained artificial intelligence may be capable of supporting a future treatment planning. At present, it may not always be possible to retrieve data sets from an existing data storage such that an efficient training of a neural network is facilitated at least due to the aforementioned drawbacks. More specifically, in some cases, it may be required to manually export patient data from a database which patient data shall be used for training an artificial intelligence and/or to track the evolution of radiotherapy treatment settings. This may, e.g., be due to the fact that some database systems do not possess a structured query language (SQL) interface for automatically querying desired data sets. This approach is generally cumbersome, time consuming, requires a lot of manual input and may additionally require a conversion of the exported data into a format that is usable for the training of the artificial intelligence.

In an example, a person, e.g., a planner of radiotherapy treatment plans with data analysis expertise, coding skills and access rights to clinical databases can, in principle, extract information from databases and create reports related to radiotherapy treatment plans with their own tools. For instance, he/she could write a script which allows the evaluation of e.g. all treatment plans for a tumor site. For instance, this could result in the mean OAR dose for each head and neck OARs averaged over a half year (+/-SD), shown for the last 10 years. Or the mean PTV dose and target coverage, e.g., a dose received by a certain percentage of the PTV. This type of information could indicate if changes in the planning process have led to only improvements in the radiotherapy treatment planning or also to a worsening of plans. However, it requires special and dedicated skills and intensive training of the staff carrying out these tasks.

Therefore, there is a need to further improve the providing of patient related data from a data storage.

It is one object to improve the tailored retrieval of radiotherapy treatment related data from a data storage.

According to a first aspect, a computer-implemented method for analyzing a plurality of radiotherapy treatment plans is suggested. The computer-implemented method comprises defining a search criterion for identifying the plurality of radiotherapy treatment plans in a data storage, defining a clinical metric to be analyzed based on the identified plurality of radiotherapy treatment plans, and requesting, from the data storage, information associated with the plurality of radiotherapy treatment plans according to the defined search criterion. Moreover, the computer-implemented method may comprise retrieving, from the data storage, the information according to the request, analyzing the received information according to the defined clinical metric and outputting a result of the analysis of the information.

The computer-implemented method may be executed on a personal computer and/or on a server and/or in a virtual environment (e.g., in a cloud environment).

A radiotherapy treatment plan may be referred to as a plan that defines a radiotherapy treatment (e.g., as a radiotherapy treatment plan for cancer cells) for a patient, and the concerning dose distribution. In some examples, the radiotherapy treatment plan may contain (detailed) machine instructions which may be needed in order to deliver a certain (radiation) dose distribution that was approved by a physician (e.g., an oncologist) to a patient.

The search criterion may be referred to as a parameter, or a combination of parameters that associates radiotherapy treatment plans, stored in the data storage, with a certain property by means of which the information associated with the radiotherapy treatment plan may be retrieved from a data storage. That is, the search criterion may effectively (logically) link the information to one or more radiotherapy treatment plans.

The clinical metric may be referred to as a metric which may be calculated, derived and/or extracted (e.g., if no further calculation step is required to obtain the clinical metric) from the information associated with the radiotherapy treatment plans identified by the search criterion.

The data storage may, e.g., be provided as a hospital information system. In some cases, the data storage may be provided as a database.

The retrieving of the information associated with the plurality of radiotherapy treatment plans may comprise retrieving the information associated with the plurality of radiotherapy treatment plans over a local network and/or the internet.

A radiotherapy treatment plan may comprise information on a single irradiation of cancer cells. In some examples, a radiotherapy treatment plan may comprise information on a plurality of irradiations of cancer cells.

The plurality of radiotherapy treatment plans may be related to a single patient. Alternatively, the plurality of radiotherapy treatment plans may be related to at least two patients. In some examples, the plurality of radiotherapy treatment plans may be related to a cohort of patients.

The outputting of the result of the analysis may comprise an outputting of the result of the analysis on a (touch sensitive) screen, as a spoken sequence or as a combination thereof.

In some examples, it may be possible that only a single radiotherapy treatment plan is analyzed.

In some examples, the information associated with the plurality of radiotherapy treatment plans may refer to one or more parameters (e.g., a duration of a radiotherapy, an OAR dose, etc.) which are comprised by a radiotherapy treatment plan. In some examples, the information associated with the plurality of radiotherapy treatment plans may refer to an entire radiotherapy treatment plan, i.e., the entirety of parameters (i.e., all parameters) comprised by a radiotherapy treatment plan.

This may facilitate an efficient, automated retrieval and analysis of radiotherapy treatment plans without the need for preparing a dedicated script, e.g., by a planner, for retrieving and analysis of the respective treatment plans. Therefore, the workflow of retrieving and analyzing radiotherapy treatment plans may efficiently be improved and smoothed. That is, the training efforts required for personal initiating the computer-implemented method may be decreased and simplified and thus opened to a broader spectrum of stakeholders. This may support an improved quality of radiotherapy treatments as changes of radiotherapy plans over the course of time may be tracked and analyzed.

In an embodiment, the computer-implemented method may further comprise providing a radiotherapy apparatus configuration for controlling a radiotherapy apparatus adapted to provide a radiotherapy process to the patient based on the outputting of the result of the analysis of the information.

The radiotherapy apparatus configuration may be provided as a file. The file may comprise at least one value of a parameter the radiotherapy apparatus may be set to (e.g., a maximum dose rate to be supplied by the radiotherapy apparatus and/or a radiotherapy time (e.g., during which an irradiation of malicious tumor cells occurs) and/or a gantry angle, etc.).

In some examples, the radiotherapy apparatus configuration may directly be transferred to the radiotherapy apparatus and applied by the radiotherapy apparatus. In some examples, the radiotherapy apparatus configuration may be stored at a control unit (e.g., a personal computer, a server, etc.), in a database, etc. and may subsequently be transferred to the radiotherapy apparatus.

The radiotherapy apparatus configuration may be configured such that upon applying the configuration by the radiotherapy apparatus, a radiotherapy treatment quality (e.g., with respect to a sharpened delimitation of the boundaries of a region of the tissue of a patient to be irradiated, a more precise dose delivery to a certain region of interest) of a radiotherapy treatment to be supplied by the radiotherapy apparatus may be improved. This may further facilitate a tailoring of subsequent radiotherapy treatment plans to the result of the analyzing of the received plurality of radiotherapy treatment plans.

In a further embodiment, the search criterion may comprise a treatment type, a treatment technique, a clinical site, clinical sub-site, a tumor stage, with or without nodal involvement, a minimum tumor size, a maximum tumor size, a planner ID, a gender of a patient, an age of the patient, a weight of the patient, a size of the patient, a dose prescription and/or a treatment intent template.

In some examples, the treatment type may be referred to as external beam radiotherapy, brachytherapy, electron irradiation, photon irradiation, proton irradiation or another suitable treatment type.

In some examples, the treatment technique may be referred to as, but not limited to, a 3D conformal treatment, static electron treatment, intensity modulated radiotherapy treatment, volumetric modulated arc treatment, intensity modulated proton treatment or another suitable treatment technique.

In some examples, the clinical site may refer to, e.g., a pelvis, head & neck or another clinical site. A clinical sub-site of e.g. head & neck could be oropharynx, larynx or another clinical subsite.

In some examples, the tumor stage may refer to e.g. stage X n Y m Z, where X is the stage of the primary tumor, Y is the stage of the nodal involvement and Z is referring to the number of metastases.

In some examples, a maximum tumor size may refer to the largest diameter of the tumor to be irradiated by means of a radiotherapy to be planned.

In some examples, a planner ID may refer to an identification number of a planner which may uniquely and unambiguously be assigned to the planner.

A dose prescription template may refer to the dosimetric intent of the treatment plan the clinician prescribed for the patient. The dose prescription template may comprise a prescription level for each target, a type of a trade-off chosen for the patient, an OAR protection level (e.g., indicated for instance by specifying certain values for the clinical goals or optimization objectives, etc.).

A treatment intent may refer to a palliative, curative or radical intent.

This may advantageously support a tailored retrieval of radiotherapy treatment plans of interest.

In a further embodiment, the clinical metric may comprise a dose coverage of targets, a planning target volume, a mean dose to specified organs-at-risk, a maximum dose to be delivered to an organ-at-risk, a volume of organs, a dose-volume value defined at a specific volume and/or a volume-dose value defined at a specific dose.

In some examples, a dose coverage of targets may be referred to as a (cumulative) dose that is delivered to a target. In some examples, a target may be referred to as a volume in the body of the patient that is to be irradiated.

A planning target volume may be referred to as a volume in the body of the patient that is to be irradiated.

A mean dose to specified organs-at-risk may be referred to as an average dose that is assigned to organs-at-risk in scheduled treatment plans of interest.

In some examples, a dose-to-volume value and/or a volume-to-dose value may indicate the dose level desired to be achieved for a certain percentage of volume of a structure and respectively the volume which should receive a certain level of dose level. For instance the planner may want to have at least 99 % of the target volume covered by the prescription level (V_{prescription} >= 0.99). Alternatively, the planner may want to use, e.g., 2.7 cc and a dose of less than or equal to of 55 Gy for a brain stem, indicating basically that it is not desired that a volume of 2.7 cc of the brain stem to receive more than 55 Gy.

This may efficiently support the tracking and characterization of parameters defined by defined radiotherapy treatment plans which may have a high impact on the success of an applied radiotherapy. Therefore, the quality of radiotherapies may efficiently and advantageously be improved.

In an embodiment, the analyzing may further comprise providing a histogram of the clinical metric for the plurality of radiotherapy plans.

In some examples, the histogram may represent a tool for representing how often a certain value of the clinical metric has been derived from the analyzed plurality of radiotherapy treatment plans. The histogram may be representative for an absolute number of how often a certain value of the clinical metric has been determined or may be representative for a relative number of how often a certain value of the clinical metric has been determined, e.g., relative to the total number of radiotherapy plans which are retrieved based on the defined search criterion.

Therefore, a tailored and efficient information retrieval of the temporal evolution of a clinical metric of interest may be facilitated which may support an improved generation of radiotherapy treatment plans.

In an embodiment, the analyzing of the received information may further comprise defining a period of time for which radiotherapy treatment plans are to be taken into consideration for the analyzing of the received information and providing a temporal evolution of the clinical metric based on the defined period of time.

The period of time may indicate a temporal interval in units of radiotherapies. Alternatively, the period of time may indicate a temporal interval in units of days, months or years.

Based thereon, a characterization of a temporal evolution of the plurality of radiotherapy treatment plans (and respective parameters comprised therein) over the course of the temporal interval may be facilitated.

In an embodiment, the defining of the search criterion and the defining of the at least one clinical metric may further comprise selecting a profile out of a plurality of pre-defined profiles for analyzing the plurality of radiotherapy treatment plans.

In some examples, the at least one search criterion and the at least one clinical metric may be comprised by a profile. In some examples, the profile may act as a template for analyzing the plurality of radiotherapy plans.

The pre-defined profiles may be pre-defined by a radiotherapy planner, a member of a medical staff, a provider of the computer-implemented method or any other capable entity.

Providing a plurality of pre-defined profiles may further support an efficient and versatile analyzing of the plurality of radiotherapy treatment plans.

The computer-implemented method may further comprise adding a new profile to the plurality of pre-defined profiles for analyzing the plurality of radiotherapy treatment plans wherein the new profile defines a combination of at least one search criterion and at least one clinical metric which is not comprised by any of the profiles in the plurality of pre-defined profiles.

In some examples, the combination of the at least one search criterion and the at least one clinical metric may be a metric which has not been part of the plurality of pre-defined profiles. In some examples, the combination of the at least one search criterion and the at least one clinical metric may be a metric which was unknown (or not-defined) at the time when the plurality of pre-defined profiles was implemented.

This may efficiently support a maintenance of analysis tools for analyzing a plurality of radiotherapy treatment plans. Moreover, it may allow a tailoring of the computer-implemented method for analyzing a plurality of radiotherapy treatment plans to circumstantial needs.

The computer-implemented method may further comprise defining a new radiotherapy treatment plan, comparing the defined new radiotherapy treatment plan to the analyzed plurality of radiotherapy treatment plans with respect to the defined clinical metric and determining, based on the comparing, whether the new radiotherapy treatment plan is in accordance with the analyzed plurality of radiotherapy treatment plans.

The new radiotherapy treatment plan may be referred to as a radiotherapy treatment plan that is not comprised in the data storage. In some examples, the new radiotherapy treatment plan may be referred to as a treatment plan that is to be used as a basis for a subsequent cancer irradiation.

The comparing may comprise a comparing whether a quality of a radiotherapy that is executed based on the new radiotherapy treatment plan is expected to be in accordance with the analyzed plurality of radiotherapy treatment plans. The new radiotherapy treatment plan may be considered as being in accordance with the analyzed plurality of radiotherapy treatment plans if a certain (predefined) mathematical relationship (e.g., between OAR dose volume histograms and PTV size, distance, etc., for each OAR) falls in the range of those determined from the analyzed plurality of radiotherapy treatment plans, while this also accounts for PTV dose volume histograms. In some examples, a new radiotherapy treatment plan may be considered as being in accordance with the analyzed information if no OAR dose can be lowered by treatment planning efforts without either increasing other OAR doses or deteriorating the dose to the PTV. In some examples, the new radiotherapy treatment plan may be considered as being in accordance with the analyzed radiotherapy treatment plans if the outcome of the radiotherapy, when executed based on the new radiotherapy treatment plan, leads to an increase of the quality of the radiotherapy.

A quality of a radiotherapy may be understood as an indicator that is representative for a success of the radiotherapy. The success may indicate a percentage of radiotherapies as a result of which a pre-defined goal has been reached. The goal may, e.g., be a devitalization of a certain volume of cancer cells. Additionally or alternatively, the quality of a radiotherapy may be based on an extent to which a target is covered during radiotherapy according to a respectively defined radiotherapy treatment plan. In some examples, the dose to OAR may define a possible toxicity which may be experienced by a patient.

Therefore, a sanity check may be facilitated which may allow an evaluation whether a new defined radiotherapy treatment plan is expected to increase a quality of a radiotherapy prior to executing the radiotherapy according to the defined new radiotherapy treatment plan. Consequently, the quality of the radiotherapy and associated healing prospects may efficiently be improved.

The computer-implemented method may further comprise verifying that the defined new radiotherapy treatment plan is to be applied for a radiotherapy of a patient if it is determined, based on the comparing, that the new radiotherapy treatment plan is in accordance with the analyzed plurality of radiotherapy treatment plans.

This may allow that the defined new radiotherapy treatment plan is only applied in response to a determining (and thus a verification) that the defined new radiotherapy treatment plan is in accordance with the analyzed plurality of radiotherapy treatment plans. Therefore, an undesired disadvantageous execution of a radiotherapy treatment plan which is not expected to support a desired success rate of the radiotherapy (e.g., if the success rate of the radiotherapy is defined as a combination of a minimum dose coverage of a target and a dose as low as possible to organs at risk) may thus not be executed and harmful effects on the patient may advantageously be avoided.

The computer-implemented method may further comprise altering at least one parameter of the new radiotherapy treatment plan if it is determined, based on the comparing, that the new radiotherapy treatment plan is not in accordance with the analyzed plurality of radiotherapy treatment plans.

In some examples, the comparing (that the new radiotherapy treatment plan is in accordance with the analyzed plurality of radiotherapy treatment plans), the determining (that the new radiotherapy treatment plan is not in accordance with the analyzed plurality of radiotherapy treatment plans) and the altering (of the at least one parameter of the new radiotherapy plan) may be executed repeatedly and/or iteratively.

This may support a tailoring of the new radiotherapy treatment plan such that, when a radiotherapy is executed according to the new radiotherapy treatment plan, a quality of the radiotherapy may be improved and thus adapted to current needs. Based on an iterative altering of the at least one parameter, an adaption of the new radiotherapy treatment plan may be facilitated such that the new radiotherapy treatment plan may effectively be tailored to support the execution of a radiotherapy which may lead to a certain expected quality of the radiotherapy. Therefore, an optimized radiotherapy may be provided to a patient.

The computer-implemented method may further comprise providing a planner of the new radiotherapy treatment plan with a training recommendation if it is determined, based on the comparing, that the new radiotherapy treatment plan is not in accordance with the analyzed plurality of radiotherapy treatment plans.

In some examples, the planner may be a member of a medical staff that is in charge of planning and (optionally) of executing the radiotherapy according to the new radiotherapy treatment plan. In some examples, the planner may be an artificial intelligence that is trained to provide boundary conditions (e.g., desired ranges for parameters associated with the radiotherapy) based on which the radiotherapy treatment plan may be planned. In some examples, the artificial intelligence may assist a human planner in planning the new radiotherapy treatment plan. In some examples, the planner in charge may have experienced extensive profession training on how to plan optimized radiotherapy treatment plans which meet the needs of a patient the best. In some examples, settings of the planning software may change, a planner may change a workplace from a first hospital or practice to a second hospital or practice wherein the second hospital or practice may use slightly different boundary conditions for conducting the planning as compared to the first hospital or practice. Consequently, the planner may not always be capable of providing a new radiotherapy plan that leads to an optimized radiotherapy for the patient.

In some cases, a training recommendation may refer to a recommendation that the planner should receive further professional training as the determining, whether the new radiotherapy treatment plan is in accordance with the analyzed plurality of radiotherapy treatment plans, has shown that the new radiotherapy plan is not in accordance with the analyzed plurality of radiotherapy treatment plans.

In some examples, the determining, based on the comparing, that the new radiotherapy treatment plan is not in accordance with the analyzed plurality of radiotherapy treatment plans may be based on a determining that that a single new radiotherapy treatment plan is not in accordance with the analyzed plurality of radiotherapy treatment plans. Based thereon, the planner may be provided with the respective training recommendation.

In some examples, more than one new radiotherapy treatment plan may be defined, i.e., at least two new radiotherapy treatment plans may be defined (e.g. each of the at least two new radiotherapy treatment plans may be defined subsequently to each other over the course of time). In such a case, the determining, based on the comparing, that the new radiotherapy treatment plan is not in accordance with the analyzed plurality of radiotherapy treatment plans may be based on a determining that the at least two new radiotherapy treatment plans are not in accordance with the analyzed plurality of radiotherapy treatment plans. That is, the at least two new radiotherapy treatment plans, when applied for a radiotherapy treatment, may lead to a therapy outcome that may be seen as not improving the radiotherapy result or as even being worse as compared to a radiotherapy result associated with the analyzed plurality of radiotherapy treatment plans. Based thereon, i.e., if it is determined that the at least two new radiotherapy treatment plans may effectively not improve the radiotherapy result, the planner may, e.g., be provided with a training recommendation. This may in particular be the case, if it is determined that the new radiotherapy treatment plans may deteriorate the (expected) result of radiotherapies that are executed based on the at least two new radiotherapy treatment plans.

Therefore, an overall improved training of planners of radiotherapy treatment plans may be facilitated and the overall quality of defined new radiotherapy treatment plans may advantageously be improved.

According to a second aspect, a computer program product is suggested. The computer program product may comprise instructions which, when the program is executed by a computer, cause the computer to carry out the computer-implemented method as described herein.

A computer program product, such as a computer program means, may for example be provided or delivered as a storage medium, such as a memory card, USB stick, CD-ROM, DVD, or in the form of a downloadable file from a server on a network. This can be done, for example, in a wireless communication network by transferring a corresponding file with the computer program product or the computer program means.

According to a third aspect, a computer-implemented apparatus for analyzing a plurality of radiotherapy treatment plans is suggested. The computer-implemented apparatus may comprise a first defining unit for defining a search criterion for identifying the plurality of radiotherapy treatment plans in a data storage, a second defining unit for defining a clinical metric to be analyzed based on the identified plurality of radiotherapy treatment plans, a requesting unit for requesting, from the data storage, the plurality of radiotherapy treatment plans according to the defined search criterion and a retrieving unit for retrieving, from the data storage, information associated with the plurality of radiotherapy treatment plans according to the request. Moreover, the computer-implemented apparatus may further comprise an analyzing unit for analyzing the received information according to the defined clinical metric and an outputting unit for outputting a result of the analysis of the information.

In some examples, the defining unit and/or the second defining unit may comprise a user interface (e.g., a (touch-sensitive) screen which may be configured to receive the search criterion (or a plurality of search criteria) and/or the clinical metric).

The respective entities, e.g. the first defining unit, the second defining unit, the requesting unit, the retrieving unit, the analyzing unit and/or the outputting unit, may be implemented in hardware and/or in software. If said entity is implemented in hardware, it may be embodied as a device, e.g. as a computer or as a processor or as a part of a system, e.g. a computer system. If said entity is implemented in software it may be embodied as a computer program product, as a function, as a routine, as a program code or as an executable object.

According to an embodiment, the computer-implemented apparatus may further comprise an execution unit for executing the computer-implemented method as described herein. Moreover, the computer-implemented apparatus may comprise a further execution unit for executing the computer program product as described herein.

The execution unit for executing the computer-implemented method or the further execution unit for executing the computer program may, e.g., comprise a processor (e.g., a CPU) and/or a graphics processing unit (GPA) and/or a tensor processing unit (TPU) and/or a field programmable gate array (FPGA).

This may support efficient execution of the steps of the method as described herein.

According to a fourth aspect, a system for analyzing a plurality of radiotherapy treatment plans is suggested. The system may comprise the computer-implemented apparatus as described herein. Moreover, the system may comprise the computer program product as described herein.

In some examples, the computer-implemented apparatus and the computer program product may be provided as a single device. Alternatively, the computer-implemented apparatus and the computer program product may be arranged as two separate devices. In the latter case the computer program product may, e.g., be arranged at a remote entity such as, e.g., a remote server, a remote database, etc.

In one or more examples, the functions described may generally be implemented in hardware, software, firmware, or any combination thereof. If implemented in software, the functions may be stored on or encoded as one or more instructions or code on a computer- readable medium. Computer-readable media includes computer storage media. Storage media may be any available media that can be accessed by a computer. By way of example, and not limitation, such computer-readable media can comprise random-access memory (RAM), read-only memory (ROM), electronically erasable programmable ROM (EEPROM), compact disk (CD) ROM (CD-ROM), or other optical disk storage, magnetic disk storage or other magnetic storage devices, or any other medium that can be used to carry or store desired program code in the form of instructions or data structures and that can be accessed by a computer. Disk and disc, as used herein, includes CD, laser disc, optical disc, digital versatile disc (DVD), and floppy disk where disks usually reproduce data magnetically, while discs reproduce data optically with lasers. Combinations of the above should also be included within the scope of computer readable media.

The embodiments and features described with reference to the apparatus of the present invention apply *mutatis mutandis* to the method of the present invention and vice versa.

Further possible implementations or alternative solutions of the invention also encompass combinations - that are not explicitly mentioned herein - of features described above or below with regard to the embodiments. The person skilled in the art may also add individual or isolated aspects and features to the most basic form of the invention.

Any embodiment of the first aspect may be combined with any embodiment of the first aspect to obtain another embodiment of the first aspect. The same applies to the further aspects.

Further embodiments, features and advantages of the present invention will become apparent from the subsequent description and dependent claims, taken in conjunction with the accompanying drawings, in which:
Figs. 1A and 1B depict diagrams showing a temporal evolution of an achieved mean dose level during a radiotherapy for a left parotid gland and for a left submandibular gland;
Figs. 2A and 2B depict diagrams showing a relationship of a frequency of patients vs. a parotid right mean dose level and a relationship between a frequency of patients and a parotid left mean dose level;
Fig. 3A depicts a diagram showing a relationship of a relative volume of an irradiated volume vs. a relative dose of a total dose;
Fig. 3B depicts a diagram showing a DVH principal component score vs. a geometric distribution principal component score.
Fig. 4 depicts a flowchart of an exemplary computer-implemented method;
Fig. 5 depicts a flowchart of an exemplary computer-implemented method;
Fig. 6 depicts an exemplary computer-implemented apparatus; and
Fig. 7 depicts an exemplary system.

In the Figures, reference numerals designate like or functionally equivalent elements, unless otherwise indicated.

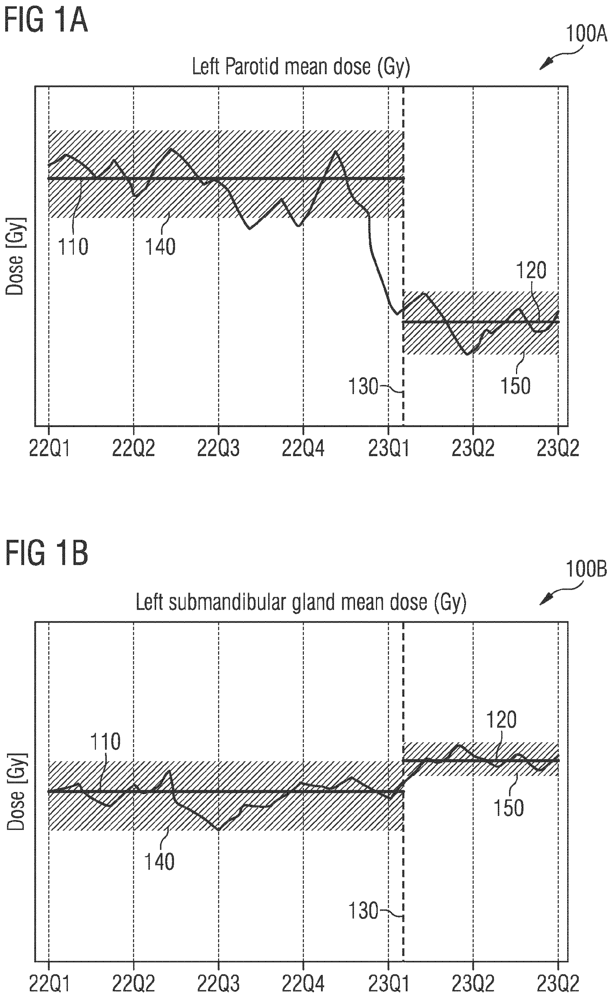

Figs. 1A and 1B depict two exemplary diagrams 100A and 100B showing a temporal evolution of an achieved mean dose level during a radiotherapy for a left parotid gland (Fig. 1A) and for a left submandibular gland (Fig. 1B).

Obtaining the diagrams 100A and 100B of Figs. 1A and 1B may be based on defining a search criterion for identifying a plurality of radiotherapy treatment plans, a defining of a clinical metric to be analyzed, a request and a retrieving of corresponding radiotherapy treatment plans in accordance with the defined search criterion and the defined clinical metric. As a result of an outputting of the result of an analysis of the plurality of received radiotherapy treatment plans, Figs. 1A and 1B may be obtained as an example.

In an example, the search criterion for retrieving the plurality of radiotherapy treatment plans may, e.g., refer to all female patients or all male patients within a certain age interval suffering from a certain type of cancer (wherein a respective tumor had been irradiated based on respective radiotherapy treatment plans) within the time span between *"22Q1"* and *"23Q2'.*

More specifically, Fig. 1A shows a temporal evolution (x-axis of Fig. 1A) of the achieved left parotid mean dose (in Gy). The x-axis indicates the temporal evolution in units of quarters of a year (e.g., the year 2022 and 2023).

In an example, the clinical metric, Fig. 1A is based on, may be defined as the *"left parotid mean dose (Gy)'.*

Based on the analyzing and the outputting of the requested plurality of radiotherapy treatment plans, it may be determined, e.g., with a view to Fig. 1A, that the left parotid mean dose (in Gy) averaged over all the selected patients in the respective period, dropped from a first mean value 110 to a second (lower) mean value 120. As derivable from diagram 100A of Fig. 1A, the first mean value 110 dropped to the second mean value 120 in the first half of the first quarter of the year 2023, indicated as the detected moment of change 130 in the diagram 100A of Fig. 1A. Based thereon, it may be derived that at least one parameter used for planning the underlying radiotherapy treatment plans has been changed such in the first half of "23Q1" that it effectively caused a drop on the mean value of the left parotid mean dose, notably, from the first mean value 110 to the second mean value 120.

As a general remark, a variation in tumor size/location may be observed between individual patients because of which a dose to OAR may vary between individual patients. Consequently, individual patients may not always be compared with each other. However, under the assumption of a patient population that may undergo a normal distribution, it may be expected that an averaging over a plurality of patients (e.g., more than 10 patients, more than 20 patients, more than 30 patients, more than 40 patients, etc.) may average out any differences between individual patients such that changes may be derivable from the obtained cohort, e.g., a mean dose to OAR, instead of from a comparison of two individual patients.

The diagram 100A of Fig. 1A further depicts the confidence interval 140 (e.g., 1 *σ*) for the first mean value 110 and the confidence interval 150 for the second mean value 120.

In analogy to the diagram 100A of Fig. 1A, the diagram 100B of Fig. 1B depicts an exemplary diagram showing the temporal evolution of the left submandibular gland mean dose (Gy). With respect to the diagram 100B of Fig. 1B, the clinical metric defined for obtaining the respective radiotherapy treatment plans referring to the left submandibular gland mean dose may thus be *"left* submandibular *gland mean dose (Gy)'.*

The generating of the diagram 100B of Fig. 1B may be identical to the generating of diagram 100A of Fig. 1A, except for the different clinical metrics defined for retrieving the respective radiotherapy treatment plans.

As derivable from Fig. 1B, the left submandibular gland mean dose (Gy) increased from a first mean value 110 to a second mean value 120 at a detected moment of change 130. The increase of the left submandibular gland mean dose occurred in the first half of *"23Q1"* as derivable from Fig. 1B. Based thereon, it may be followed that at least one parameter used for planning the underlying radiotherapy treatment plans has been changed such in the first half of *"23Q1"* that it effectively caused an increase of the mean value of the left submandibular gland mean dose, notably, from the first mean value 110 to the second mean value 120.

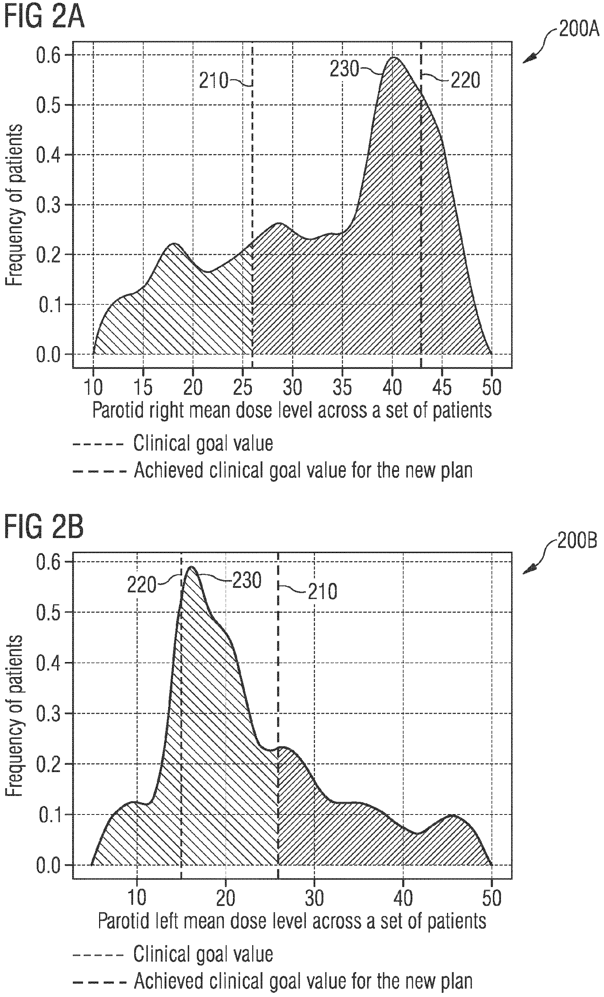

Figs. 2A and 2B depict exemplary diagrams 200A and 200B showing the relationship of frequency of patients vs. a parotid right mean dose level across a set of patients (Fig. 2A) and a parotid left mean dose level across a selected set of patients (Fig. 2B).

In both cases, a clinical goal value provided by a clinical protocol may, in an example, be 26 Gy (indicated by line 210). However, the smaller the achieved value of the radiation dose the better protection may be achieved for the irradiated tissue of the body of the patient.

Line 220 indicates an exemplary achieved clinical goal value for a new radiotherapy plan.

The *"frequency of patients"* indicates the relative amount of patients for which a certain dose level has been achieved according to the analyzed radiotherapy treatment plans.

More specifically, Fig. 2A depicts an exemplary diagram 200A representing a case in which the achieved value of the radiation dose corresponding to a new radiotherapy treatment plan does not fulfill the clinical protocol as the achieved radiation dose level is larger than 26 Gy which may also be the case in most of the historical cases (expressed by a respective plurality of radiotherapy treatment plans). This may indicate to the planner that the clinical protocol may be difficult to achieve (as the achieved dose is larger than 26 Gy in a large number of cases) and that it may be seen acceptable to approve a defined new radiotherapy treatment plan as further improvements (e.g., a lower achieved radiation dose) are unlikely to happen.

In some examples, the historical cases (expressed by the plurality of (historical) radiotherapy treatment plans) are selected to be similar to the new radiotherapy treatment plan in that the historical cases, with which the new radiotherapy treatment plan is to be compared, refer to a same metric such as, e.g., a same cancer type and/or subtype, PTV size and/or shape, target laterality, target location, distance to OAR, body size/shape, treatment type and/or intent, prescription levels, selected trade-off type etc. Based thereon, an unambiguous comparison of the new radiotherapy treatment plan to historical cases may be facilitated.

In some examples, the historical cases may be retrieved from a respective database by means of a trained deep learning algorithm. In some examples, the deep learning algorithm may have been trained to select a plurality of historical cases (e.g., a plurality of historical radiotherapy treatment plans) based on the new radiotherapy treatment plan (e.g., according to parameters associated with the new radiotherapy treatment plan and the historical cases which may be regarded similar).

In analogy to Fig. 2A, Fig. 2B depicts a diagram 200A in which the achieved clinical goal value for a new radiotherapy treatment plan is better than the clinical goal (line 210) which is also similar to what has been achieved for most historical cases. The latter assessment may, e.g., be derived from the aspect that an area between the x-axis and the curve 230 may be larger in between a parotid left mean dose level of 0 Gy and a parotid left mean dose level of 26 Gy as compared to an area between the x-axis and the curve 230 between a parotid left mean dose level of 26 Gy and 50 Gy.

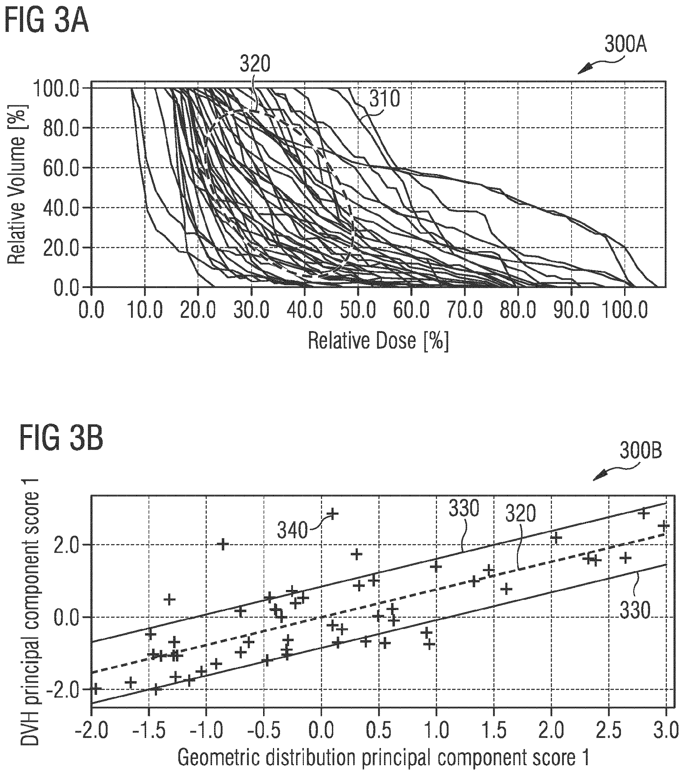

Fig. 3A depicts a diagram 300A which shows dose-volume-histograms (DVH) curves, i.e., a relationship of a relative volume (in %) of an irradiated volume in a body of a patient vs. a relative dose (in %) of a total dose that is deposited in the volume of the patient.

For generating diagram 300A, a plurality of radiotherapy treatment plans was used (each radiotherapy treatment plan being associated with a respective line in diagram 300A). Each radiotherapy plan may be associated with a respective patient, wherein the patient may be associated with a respective search criterion. The DVH curve is effectively a histogram that relates a radiation dose to a tissue volume in radiation therapy planning.

The combination of the relative dose and the relative volume may effectively describe how many percent of a total radiation dose that is achieved in a volume within a patient is achieved in a certain percentage of the total volume which is irradiated.

Line 310 may refer to an exemplary case in which a new radiotherapy treatment plan has been generated. The DVH curve for a selected structure may be derived from the new radiotherapy treatment plan and a respective combination of a relative dose and a relative volume may be calculated based on the pre-defined patient model. The result may be expressed as the line 310. Subsequently, a comparing of the defined new radiotherapy treatment plan to the analyzed plurality of historical radiotherapy treatment plans may be executed. Based on the comparing, it may be decided whether the new radiotherapy treatment plan improves the success of a radiotherapy executed accordingly. In the present case, line 310 lies significantly above those lines that are derived from historical radiotherapy treatment plans.

In some examples, the DVH may be correlated (e.g., as part of a principal component analysis) with one or more parameters related to a radiotherapy (e.g., derived from a radiotherapy treatment plan) such as, e.g., field size, field orientation, PTV size and/or shape, distance of the PTV to OAR, OAR size and/or shape. This may be advantageous when selecting cohorts of similar patients.

This may indicate that the new radiotherapy treatment plan effectively leads to a higher relative dose achieved in a higher relative volume as compared to radiotherapy outcomes that are associated with the historical radiotherapy treatment plans. This may be an indication for a planner of the new radiotherapy treatment plan that further fine tuning of the new radiotherapy treatment plan may be required to bring the outcome of a radiotherapy according to the new radiotherapy treatment plan in accordance with the outcome of radiotherapies according to historical radiotherapies (i.e. effectively shifting line 310 closer to the remaining lines indicated in the diagram 300A of Fig. 3A).

In some examples, an assessment, whether a certain new radiotherapy treatment plan may improve a respective radiotherapy that is performed according to the radiotherapy treatment plan may be based on selecting a plurality of historical radiotherapy treatment plans that are similar to the new radiotherapy treatment plan. This underlying similarity may require that the historical radiotherapy treatment plans refer to, e.g., a similar cancer type and/or subtype, treatment type, intent and/or prescription.

Fig. 3B shows a diagram 300B depicting a DVH principal component score vs. a geometric distribution principal component score.

Even though the DVH curves in Fig. 3A may not be representative for a relationship with respect to each other, a relationship may however be derived by means of extracting the principal components from the DVH curves as depicted in Fig. 3B which, e.g., depicts a relationship between the principal component of the DVH and a geometric distribution principal component.

Each of the lines depicted in diagram 300A may be transformed into individual data points depicted in diagram 300B by a mathematical process such as a principal component analysis.

As derivable from diagram 300B, the plurality of data points follows a linear trend as indicated by regression line 320 and the confidence interval 330 enclosing the regression line 320. The data points following the regression line 320 (especially within the confidence interval 330) may be considered as representing those radiotherapy treatment plans that may be lead to a desired radiotherapy quality. These radiotherapy treatment plans may be associated with an area within diagram 300A indicated by reference numeral 320 in analogy to the regression line 320.

In some examples, the principal component analysis may be replaced by a (trained) deep learning algorithm that may learn from a plurality of patients how the dose distribution is related to features of the respective patients (such as, e.g., tumor size, shape, distance to OAR, etc.).

Data point 340, as indicated in diagram 300B, corresponds to line 310 of diagram 300A of Fig. 3A which was associated with a new radiotherapy treatment plan. Also, in diagram 300B of Fig. 3B, data point 340 appears as not being in accordance with the remaining data points that are derived from historical radiotherapy treatment plans.

This may again provide a planner of the new radiotherapy treatment plan with an indication that further fine tuning of at least one parameter of the new radiotherapy treatment plan may be recommended and/or necessary.

There are several tasks in the radiotherapy treatment planning process for which data-driven approaches, e.g., using machine learning (ML) methods, could be used to improve the quality and reproducibility of patient care and at the same time decrease the variability in treating standard cases. Typical examples of such steps include organ and target segmentation and 3D dose prediction. The current state-of-the-art data-driven approaches for such tasks follow, in general, a supervised learning scheme in which a mathematical model (most often a deep neural network) is trained on a dataset containing both the input data as well as the output data (the ground truth). The OAR/PTV contours are very often used either as the input or as the output data for such models. The training process in this framework may use an optimization algorithm that searches through the space of possible values of the model weights to find a set of weights that minimizes a loss function measuring how different the current model predictions are compared with the ground truth.

The availability of OAR and PTV contours becomes a crucial element enabling the training of these mathematical models. Moreover, a systematic labeling of these contours is another essential factor enabling us to select both a suitable set of patient data which could be used for training a model as well as to identify for the model which contours from the patient's structure file to map to the structures of interest.

In some examples, the parameters which define a radiotherapy treatment plan (e.g., effectively expressed as an answer to a requested search criterion and/or a clinical metric) may be phrased ambiguously. This may, e.g., affect the labeling of a clinical metric as such and/or its value (and/or of a search criterion). That is, a textual description of, e.g., OARs as well as for target structures (to be irradiated) and/or any other parameters that are associated with the planning of a radiotherapy may underly a variation in the naming styles. This may be due to differences in spelling of different languages (e.g., in different countries, different clinics, etc.) from which radiotherapy treatment plans may be retrieved. In an example, labels used to describe *"parotid glands"* may, e.g., be phrased as *"Parotid_L", "parotid gland 1", "left parotid', "Parotid_gl_left"* and *"Parotid_LT"* even though they effectively describe the same organ. Moreover, different languages based on which the parameters of the radiotherapy treatment plan may be labeled may additionally lead to differences in the spelling. In an example, the terms *"Parotid G'* (derived from parotid gauche in French) and *"Parotid D"* (derived from parotid droit in French) may be used to describe directions associated with the parotid such as, e.g., in a French clinic. Additionally or alternatively, organs may also be described by using structure codes.

Structure codes may refer to unique IDs which are associated with structures, thus eliminating the problems related to the variation in the labels. Structure codes are, however, not always used.

In case a systematic use of the structure codes for all structures applies, an advantageous identification of the contours associated with specific structures across a cohort of patient cases may be facilitated, independent of the language used or variations in naming conventions.

Regarding the definition of a search criterion, it may be advantageous to provide a systematic labeling scheme for the patient selection, which may be seen as being of particular importance whenever a subset of patient data is to be selected from a large database/data satisfying certain criteria. For instance, an initial filtering to select prostate radiotherapy treatment plans could be done by checking if the patient structure files (which may refer to a standard file type in radiotherapy which may comprise patient's geometric information) contain structured contours (e.g., numerical definitions of outlines of structures (e.g., prostate, bladder, etc.) which may be defined in a two-dimensional plane) for prostate, bladder, or rectum. Alternatively, if an autosegmentation model shall be trained for a certain set of structures, their labels can be used to select a suitable patient data training set. In some examples, a further filtering using certain criteria (e.g. whether the plans were curative or palliative, the used field geometry, specific OAR sparing-target coverage trade-offs) may be applied. However, in order to be able to perform any of these filtering steps, a systematic labeling schema (the same fixed label for a given structure, independent of the clinic of origin for the treatment plan) may be required.

Therefore, it may be advantageous to automatically curate the labels of manually drawn contours from the patients' raw data and establish a naming standard. This, in turn, will make the data selection and data processing easier for the subsequent models trained and used for various radiotherapy treatment planning tasks. This may, e.g., be due to a trained autosegmentation algorithm, trained for a predefined set of structures. In some examples, this may be extended to a case of several autosegmentation algorithms, each trained for a different set of structures.

In an example, it may be required to identify (and curate the labels of) both parotid glands for a large set of patients. For that purpose, it may be assumed that an autosegmentation algorithm is trained to generate contours for both the left and the right parotid glands, labeled as *"Parotid_L"* and *"Parotid_R',* respectively. Next, this algorithm may be applied on the whole set of patients wherein these two contours may be generated for each patient. Then, these generated contours may be compared against all structures from the patient's raw structure file and the similarity of the contour pairs may be evaluated, e.g., by computing Dice scores between the automatically generated contour and each of the manually drawn contours of the patient. Then, from the patient's raw file, the structure for which the largest Dice score is obtained as the natural match for this structure, a respective label may be selected, i.e., by renaming it with a standard label.

There might be some cases in which there are several manual contours related to a certain structure, e.g., spinal cord and spinal cord plus a margin, or parotid gland and parotid gland minus the part overlapping the target. In such cases, a non-zero (and maybe quite high) Dice score may be obtained for all these manual contours.

One possible approach in such a case would be to compute the volumes of these manual structures and to then make the structure selection using user-defined decision rules. For instance, a planner could decide to select the structure with the larger volume among the parotid gland and parotid gland minus the part overlapping the target.

For some organs it may be possible that only part of the organ has been contoured (for example in the case of spine, esophagus, or lungs). For a more robust result it may be possible to restrict the Dice score calculation only to those (CT) layers where an organ is defined. If the auto-segmentation model provides a fully contoured organ, this approach may still lead to a high Dice score.

If the method is simultaneously applied to a batch of plans, one could combine the proposed method to also exclude plans based on the names: for example, if an organ named *"spine+2mm"* is having highest score with automatically generated structure *"spine"* but all the other *"spine+2mm"* named structures are having highest score with automatically generated structure *"spine-PREV"* the organ should be mapped to the latter.

It may also be possible to provide confidence information in addition to the mapping in cases where the best dice score is not very high (or there are two matches with quite similar Dice score). Alternatively, it may be possible to give multiple matches with varying (approximated) probability if such information is desired.

In some cases, the autosegmentation algorithm may be developed in such a way that it returns both structure contours as well as a confidence value for these predictions (or uncertainty value).

In those cases, the confidence values of the predictions can be used to decide whether there is a match with a manually drawn contour. That is, if the model confidence values for a predicted contour are very low, it may be decided to skip entirely the matching with the manually drawn contours and to consider that none of these are a match for the structure of interest.

Additionally, if no structure is found in the set of manually contoured structures that matches the autosegmented one well enough, i.e. with high enough Dice score, the planner might decide to add the automatically generated structure as the desired structure. With this approach, the process would produce more valid data for machine learning training projects compared to just skipping the nonmatching structures. This may be done with the user's review, possible correction, and approval.

Therefore, a method to automatically curate OARs/PTVs labels is proposed using an already trained autosegmentation model for the selected OARs/PTVs. This may be based on a trained autosegmentation model to predict contours for a selected set of structures. Then, evaluation metrics, e.g. Dice score, may be used to compare the automatically generated contour of a structure with all structures from a raw structure file of each patient. Assuming a robust, and accurate autosegmentation algorithm, for a given structure, the manually drawn contour which achieves the highest value of the selected metric when compared with the automatically generated contour will be identified as the right contour for that structure. In that case a relabeling of the identified manually drawn contour with a chosen standard label may be facilitated which can then be used for the training of other models (e.g., 3D dose prediction, outcome prediction models) or when filtering data based on structure labels.

It is emphasized that even though aspects of the invention have been described with reference to parotid glands, the application of the aspects described herein are not limited to parotid glands only but may also be applied to other organs not expressly mentioned herein.

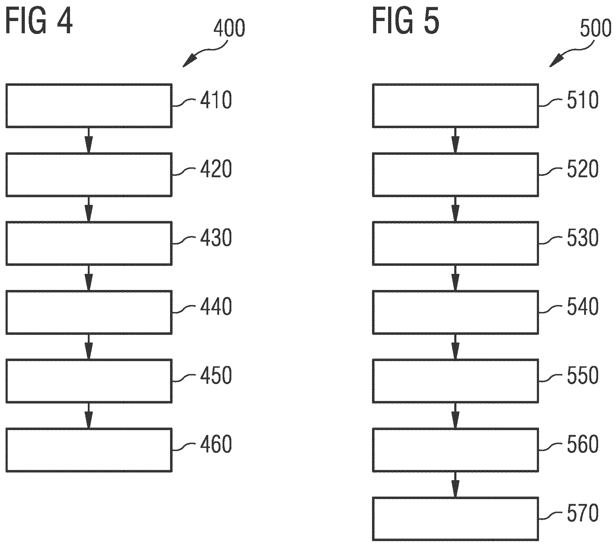

Fig. 4 shows an exemplary computer-implemented method 400 for analyzing a plurality of radiotherapy treatment plans.

In step 410, a search criterion for identifying the plurality of radiotherapy treatment plans in a data storage is performed.

In step 420, a clinical metric to be analyzed based on the identified plurality of radiotherapy treatment plans is defined.

In step 430, the plurality of radiotherapy treatment plans according to the defined search criterion is requested from the data storage.

In step 440, information associated with the plurality of radiotherapy treatment plans is retrieved according to the request.

In step 450, the received information is analyzed according to the defined clinical metric.

In step 460, a result of the analysis of the information is output.

Fig. 5 shows an exemplary computer-implemented method 500 for analyzing a plurality of radiotherapy treatment plans.

Steps 510 - 560 of the computer-implemented method 500 are identical to steps 410 - 460 of computer implemented method 400 as described above.

Computer-implemented method 500 further comprises step 570.

In step 570, a radiotherapy apparatus configuration is provided for controlling a radiotherapy apparatus adapted to provide a radiotherapy process to the patient based on the outputting of the result of the analysis of the plurality of radiotherapy treatment plans.

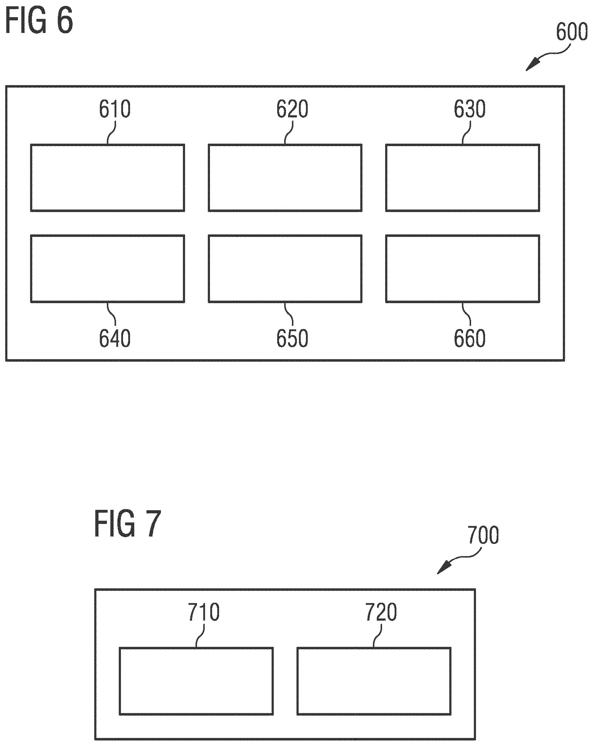

Fig. 6 depicts an exemplary computer-implemented apparatus 600. The computer-implemented apparatus 600 comprises a first defining unit 610, a second defining unit 620, a requesting unit 630, a retrieving unit 640, an analyzing unit 650 and an outputting unit 660.

The first defining unit 610 is configured for defining a search criterion for identifying the plurality of radiotherapy treatment plans in a data storage.

The second defining unit 620 is configured for defining a clinical metric to be analyzed based on the identified plurality of radiotherapy treatment plans.

The requesting unit 630 is configured for requesting, from the data storage, the plurality of radiotherapy treatment plans according to the defined search criterion.

The retrieving unit 640 is configured for retrieving, from the data storage, information associated with the plurality of radiotherapy treatment plans according to the request.

The analyzing unit 650 is configured for analyzing the received information according to the defined clinical metric.

The outputting unit 660 is configured for outputting a result of the analysis of the information.

Fig. 7 shows an exemplary system 700 for analyzing a plurality of radiotherapy treatment plans. The system 700 may include a computer-implemented apparatus 710 and a computer program product 720.

Computer-implemented apparatus 710 may be configured as the computer-implemented apparatus 600.

Computer program product 720 may be configured as described elsewhere herein.

Although the present invention has been described in accordance with preferred embodiments, it is obvious for the person skilled in the art that modifications are possible in all embodiments.

Independent of the grammatical term usage, individuals with male, female or other gender identifies are included with the term.

### LIST OF REFERENCE

- 100A: diagram
- 100B: diagram
- 110: first mean value
- 120: second mean value
- 130: moment of change
- 140: confidence interval
- 150: confidence interval
- 200A: diagram
- 200B: diagram
- 210: line
- 220: line
- 230: curve
- 300A: diagram
- 300B: diagram
- 310: line
- 320: line
- 330: confidence interval
- 340: data point
- 400: computer-implemented method
- 410: step
- 420: step
- 430: step
- 440: step
- 450: step
- 460: step
- 500: computer-implemented method
- 510: step
- 520: step
- 530: step
- 540: step
- 550: step
- 560: step
- 570: step
- 600: computer-implemented apparatus
- 610: first defining unit
- 620: second defining unit
- 630: requesting unit
- 640: retrieving unit
- 650: analyzing unit
- 660: outputting unit
- 700: system
- 710: computer-implemented apparatus
- 720: computer program product

## Claims

1. A computer-implemented method (400; 500) for analyzing a plurality of radiotherapy treatment plans, comprising:
defining (410; 510) a search criterion for identifying the plurality of radiotherapy treatment plans in a data storage;
defining (420; 520) a clinical metric to be analyzed based on the identified plurality of radiotherapy treatment plans;
requesting (430; 530), from the data storage, the plurality of radiotherapy treatment plans according to the defined search criterion;
retrieving (440; 540), from the data storage, information associated with the plurality of radiotherapy treatment plans according to the request;
analyzing (450; 550) the received information according to the defined clinical metric; and
outputting (460; 560) a result of the analysis of the information.

2. The computer-implemented method (500) of claim 1, further comprising:
providing (570) a radiotherapy apparatus configuration for controlling a radiotherapy apparatus adapted to provide a radiotherapy process to the patient based on the outputting of the result of the analysis of the information.

3. The computer-implemented method of one of the claims 1 or 2,
wherein the search criterion comprises a treatment type, a treatment technique, a clinical site, a minimum tumor size, a maximum tumor size, a planner ID, a gender of a patient, an age of the patient, a weight of the patient, a size of the patient and/or a treatment intent template.

4. The computer-implemented method of one of the preceding claims,
wherein the clinical metric comprises a dose coverage of targets, a planning target volume, a mean dose to specified organs-at-risk, a maximum dose to be delivered to an organ-at-risk, a volume of organs, dose-volume value defined at a specific volume and/or a volume-dose value defined at a specific dose.

5. The computer-implemented method of one of the preceding claims, wherein the analyzing further comprises:
providing a histogram of the clinical metric for the plurality of radiotherapy plans.

6. The computer-implemented method of one of the preceding claims, wherein the analyzing of the received information further comprises:
defining a period of time for which radiotherapy treatment plans are to be taken into consideration for the analyzing of the received information; and
providing a temporal evolution of the clinical metric based on the defined period of time.

7. The computer-implemented method of one of the preceding claims, wherein the defining of the search criterion and the defining of the at least one clinical metric further comprises:
selecting a profile out of a plurality of pre-defined profiles for analyzing the plurality of radiotherapy treatment plans.

8. The computer-implemented method of claim 7, further comprising:
adding a new profile to the plurality of pre-defined profiles for analyzing the plurality of radiotherapy treatment plans wherein the new profile defines a combination of at least one search criterion and at least one clinical metric which is not comprised by any of the profiles in the plurality of pre-defined profiles.

9. The computer-implemented method of one of the preceding claims, further comprising:
defining a new radiotherapy treatment plan;
comparing the defined new radiotherapy treatment plan to the analyzed plurality of radiotherapy treatment plans with respect to the defined clinical metric; and
determining, based on the comparing, whether the new radiotherapy treatment plan is in accordance with the analyzed plurality of radiotherapy treatment plans.

10. The computer-implemented method of claim 9, further comprising:
verifying that the defined new radiotherapy treatment plan is to be applied for a radiotherapy of a patient if it is determined, based on the comparing, that the new radiotherapy treatment plan is in accordance with the analyzed plurality of radiotherapy treatment plans.

11. The computer-implement method of claim 10, further comprising:
altering at least one parameter of the new radiotherapy treatment plan if it is determined, based on the comparing, that the new radiotherapy treatment plan is not in accordance with the analyzed plurality of radiotherapy treatment plans.

12. The computer-implemented method of one of the claims 9 or 11, further comprising:
providing a planner of the new radiotherapy treatment plan with a training recommendation if it is determined, based on the comparing, that the new radiotherapy treatment plan is not in accordance with the analyzed plurality of radiotherapy treatment plans.

13. A computer program product comprising instructions which, when the computer program product is executed by a computer, cause the computer to carry out the computer-implemented method according to one of the claims 1-12.

14. A computer-implemented apparatus (600) for analyzing a plurality of radiotherapy treatment plans, comprising:
a first defining unit (610) for defining a search criterion for identifying the plurality of radiotherapy treatment plans in a data storage;
a second defining unit (620) for defining a clinical metric to be analyzed based on the identified plurality of radiotherapy treatment plans;
a requesting unit (630) for requesting, from the data storage, the plurality of radiotherapy treatment plans according to the defined search criterion;
a retrieving unit (640) for retrieving, from the data storage, information associated with the plurality of radiotherapy treatment plans according to the request;
an analyzing unit (650) for analyzing the received information according to the defined clinical metric; and
an outputting unit (660) for outputting a result of the analysis of the information.

15. The computer-implemented apparatus of claim 14, further comprising:
an execution unit for executing the computer-implemented method according to one of the claims 1-12; and/or
a further execution unit for executing the computer program product according to claim 13.
